(19) Europäisches Patentamt
European Patent Office
Office européen des brevets

(11) EP 0 601 113 B1

(12) FASCICULE DE BREVET EUROPEEN

(45) Date de publication et mention de la délivrance du brevet:
**08.01.1997 Bulletin 1997/02**

(21) Numéro de dépôt: **92919653.3**

(22) Date de dépôt: **28.08.1992**

(51) Int Cl.⁶: **C07F 5/00**, G01N 33/52, G01N 33/533, G01N 33/542

(86) Numéro de dépôt international:
**PCT/FR92/00832**

(87) Numéro de publication internationale:
**WO 93/05049 (18.03.1993 Gazette 1993/08)**

# (54) COMPLEXES MACROCYCLIQUES DE TERRES RARES ET LEUR UTILISATION POUR REDUIRE LES INTERFERENCES DANS UN DOSAGE PAR FLUORESCENCE

Makrozyklische Seltener-Erden-Komplexe und deren Verwendung für die Reduzierung von Interferenzen in Fluoreszenzbestimmungsverfahren

RARE EARTH MACROCYCLIC COMPLEXES AND USE THEREOF FOR REDUCTION OF DISTURBANCES IN AN ASSAY USING FLUORESCENCE

(84) Etats contractants désignés:
**AT BE CH DE DK ES FR GB GR IE IT LI LU MC NL SE**

(30) Priorité: **30.08.1991 FR 9110809**

(43) Date de publication de la demande:
**15.06.1994 Bulletin 1994/24**

(73) Titulaire: **CIS BIO INTERNATIONAL**
**F-91000 Saclay (FR)**

(72) Inventeurs:
- **LEHN, Jean-Marie**
  **F-67000 Strasbourg (FR)**
- **ROTH, Christine, Odile**
  **F-67000 Strasbourg (FR)**
- **MATHIS, Gérard**
  **F-30200 Bagnols-sur-Cèze (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 180 492** **EP-A- 0 195 413**
**WO-A-89/08263**

- **CHEMICAL ABSTRACTS, vol. 115, 1991, Columbus, Ohio, US; abstract no. 122859a, PRODI, L. ET AL 'LUMINESCENCE PROPERTIES OF CRYPTATE EUROPIUM(III) COMPLEXES INCORPORATING HETEROCYCLIC N-OXIDE GROUPS' page 558 ;**
- **HELVETICA CHIMICA ACTA vol. 74, no. 3, 2 Mai 1991, pages 572 - 578 LEHN, J.-M.**
- **ET AL '56. SYNTHESIS AND PROPERTIES OF SODIUM AND EUROPIUM(III) CRYPTATES INCORPORATING THE 2,2'-BIPYRIDINE 1,1'-DIOXIDE AND 3,3'-BIISOQUINOLINE 2,2'-DIOXIDE UNITS' cité dans la demande**
- **HELVETICA CHIMICA ACTA vol. 73, no. 1, 31 Janvier 1990, pages 106 - 111 LEHN, J.-M. ET AL '10. SYNTHESIS AND PROPERTIES OF ACYCLIC AND CRYPTATE EUROPIUM(II) COMPLEXES INCORPORATING THE 3,3'-BIISOQUINOLINE 2,2'-DIOXIDE UNIT' cité dans la demande**
- **CHEMICAL ABSTRACTS, vol. 115, 1991, Columbus, Ohio, US; abstract no. 122859a, PRODI, L. ET AL 'LUMINESCENCE PROPERTIES OF CRYPTATE EUROPIUM(III) COMPLEXES INCORPORATING HETEROCYCLIC N-OXIDE GROUPS' page 558 ;**
- **Chem. Phys. Lett. 1991, 180,45-50 .**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 601 113 B1

Printed by Jouve, 75001 PARIS (FR)

## Description

L'invention concerne des complexes macrocycliques de terres rares, un procédé de réduction d'interférences dans un dosage par fluorescence utilisant ces complexes, ainsi que leur utilisation pour réduire les interférences dans le milieu de mesure d'un procédé de détection et/ou de détermination par fluorescence d'un analyte dans un milieu susceptible de le contenir.

L'utilisation de dosages immunologiques pour l'analyse qualitative et quantitative de composés dans des fluides biologiques est à l'heure actuelle largement répandue.

Parmi les techniques existantes, les dosages par fluorimétrie ont pris une importance croissante.

En effet, ils présentent un certain nombre d'avantages parmi lesquels la sensibilité, la rapidité de la mesure, la stabilité et l'inocuité des réactifs marqués par des composés fluorescents et le coût relativement réduit.

Il est connu que les méthodes de détection utilisant la fluorescence sont intrinsèquement très sensibles et pourraient permettre des limites de détection inférieures à celles atteintes par des dosages immunologiques utilisant des réactifs radiomarqués, en particulier par l'utilisation de sources lumineuses modulables laser (I. Wieder, Immunofluorescence and related staining techniques, 1978, Elsevier).

De nombreuses molécules fluorescentes utilisables comme traceur dans ce type de dosages ont été précédemment décrites parmi lesquelles les complexes de terre rare possèdent des propriétés intéressantes.

L'utilisation de complexes particuliers, les cryptates de terre rare, est décrite dans les demandes EP 0 180 492, PCT/FR 86 00269, EP 0 321 353 ou PCT/FR 89 00562.

Ces cryptates de terre rare présentent l'avantage d'être très stables en milieu protéique et salin, cette propriété étant particulièrement importante dans le cas des immunoessais homogènes.

La sensibilité de la mesure peut néanmoins être fortement affectée par des interférences de différents types résultants de la présence de diverses molécules dans le milieu de mesure.

Ce problème se pose de manière particulièrement aiguë dans le cas de dosages en milieu sérique dans lequel de nombreuses molécules sont susceptibles d'interférer.

Le signal mesuré peut par exemple être parasité par l'émission de molécules susceptibles d'être excitées et d'émettre aux mêmes longueurs d'onde que la molécule utilisée comme traceur.

Les méthodes de mesure de fluorescence en temps résolu permettent de remédier partiellement à cet inconvénient. Le principe de ces méthodes réside dans le fait qu'on effectue la mesure de la fluorescence émise par une molécule traceur ayant une durée de vie d'émission relativement longue, la mesure étant retardée dans le temps audelà de la durée de vie d'émission des autres molécules présentes.

Il est dans ce cas nécessaire d'utiliser des molécules fluorescentes traceurs à durée de vie relativement longue telles que les chélates de terre rare.

Ainsi, l'article de Prodi et al. dans Chem. Phys. Letters, 1991, 180, 45-50 décrivent les propriétés luminescentes de certains cryptates ou complexes macrocycliques d'europium.

La sensibilité de la mesure peut également être affectée par l'interférence de molécules du milieu susceptible de perturber la variation de fluorescence résultant de la liaison entre l'analyte à détecter et le réactif biospécifique marqué. La demande EP 0 324 323 décrit l'utilisation d'un modulateur qui stabilise le chélate de terre rare lié au réactif biospécifique, de telle sorte que la fluorescence mesurée soit réellement fonction de la concentration de l'analyte. Ce modulateur a pour effet d'éviter la perturbation de la fluorescence du chélate de terre rare par les autres molécules présentes dans le milieu. La variation de fluorescence mesurée est alors fonction de la seule réaction antigène-anticorps. Les modulateurs proposés sont des macromolécules telles que des protéines et des détergents, et doivent être utilisées en excès dans la gamme de 0,1 à 10 g/l.

Le problème des interférences dues aux molécules présentes dans le milieu de mesure n'est néanmoins complètement résolu par aucune de ces méthodes. En effet, une source importante de limitation de la sensibilité de la mesure par fluorescence est l'existence de processus d'extinction ("quenching") dus à des molécules présentes dans le milieu capables d'inhiber la fluorescence de la molécule fluorescente utilisée comme marqueur dans le dosage. Dans le cas des complexes de terre rares, ces processus peuvent résulter de mécanismes de transfert d'électrons par proximité, dans lesquels la molécule inhibitrice vient occuper les sites de coordination restés libres au sein du complexe. On peut en particulier citer les réactions red/ox intervenant entre la molécule fluorescente dans son état fondamental ou dans son état excité et des molécules présentes dans le milieu. Ces mécanismes sont susceptibles de faire varier de façon non négligeable la fluorescence émise.

L'article Weber et al, Clin. Chem, 1983, 29/9, 1665-1672, décrit l'influence d'interférences dues en particulier à l'acide urique dans la détection ampérométrique d'un complexe Tris(2',2'-bipyridine)ruthénium (III). Ce complexe provient de la réaction red/ox du complexe correspondant Ru(II) capable d'oxyder un complexe extincteur Co(III). L'acide urique a été identifié en tant qu'agent réducteur de Ru(III) et est donc susceptible d'interférer dans la mesure.

Ce mécanisme red/ox dû à un transfert d'électron entre un composé fluorescent et un composé extincteur a également été mis en évidence par Sabbatini et al., J.A.C.S., 1984, 106, 4055-4056. Cet article décrit notamment l'oxydation d'un complexe $M(CN)_6^{4-}$ dans lequel M est le fer, le ruthénium ou l'osmium par un cryptate d'europium.

L'inhibition de la fluorescence par des mécanismes mettant en jeu un transfert d'électrons et en général par des mécanismes d'extinction est un phénomène extrêmement gênant dans la pratique car les éléments inhibiteurs peuvent soit se trouver naturellement présents en tant que composants dans le milieu de mesure (par exemple l'acide urique dans le sérum) ou encore y être ajoutés comme additifs ou stabilisants pour le dosage.

Ces inhibiteurs affectent fortement la fluorescence de la molécule marqueur. En particulier, dans le cas des réactions parasites red/ox le passage de l'état réduit à l'état oxydé d'un ion de terre rare par le biais d'un mécanisme red/ox entraîne une diminution de la durée de vie et une modification de spectre d'émission du complexe le contenant, de telle sorte que la sensibilité de la mesure est fortement affectée.

L'utilisation de ligands de type macrocycles pour chélater les ions de terres rares a été décrite dans la littérature, notamment dans les publications suivantes : J. Phys. Chem, 1987, 91, 4681-4685, Inorg. Chem. 1983, 22, 3866-3869, Inorg. Chem. Acta 1984, 95n 119-125, Nucl. Med. Biol. 1986, 13 311-318, Comprehensive Coordination Chemistry 1987 Vol. 3, Ed. Pergamon Press., ainsi que dans la publication Helvetica Chemica Acta, 1990, 73, 1149-1162.

Bien que certains des composés décrits présentent une faible vitesse de dissociation dans l'eau, le critère de stabilité dans l'eau pure n'est pas suffisant puisque le sérum contient en fait de nombreux ions et protéines dont certaines à forte concentration qui peuvent entrer en compétition avec le ligand pour la complexation de l'ion de terre rare.

De manière inattendue, on a maintenant trouvé que des complexes macrocycliques de terre rare constitués d'au moins un sel de terre rare complexé par un composé macrocyclique comprenant au moins un motif moléculaire possédant une énergie de triplet supérieure à celle du niveau émissif de l'ion de terre rare complexé et au moins un système hétérocyclique azoté dont l'un au moins des atomes d'azote porte un groupe oxy, présentent la propriété avantageuse d'être significativement moins sensibles à l'extinction de fluorescence ("quenching") due aux inhibiteurs présents dans le milieu de mesure que les chélates et les complexes macropolycycliques et macrocycliques connus et d'être plus stables en milieu biologique que les autres complexes macrocycliques et chélates connus.

Ils sont de ce fait particulièrement adaptés à une utilisation en tant que traceurs dans un dosage par fluorimétrie.

Dans un premier aspect, l'invention a donc pour objet des complexes macrocycliques de terres rares, caractérisés en ce qu'ils sont constitués d'au moins un sel de terre rare complexé par un composé macrocyclique de formule (I)

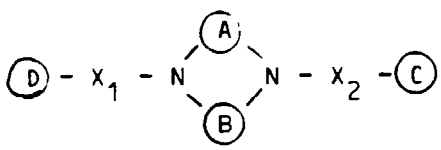

(I)

dans laquelle :

- les radicaux bivalents Ⓐ, Ⓑ, © et Ⓓ, identiques ou différents, représentent des chaînes hydrocarbonées contenant éventuellement un ou plusieurs hétéroatomes, l'un au moins desdits radicaux comportant au moins un motif moléculaire ou étant essentiellement constitué par un motif moléculaire possédant une énergie de triplet supérieure à celle du niveau émissif de l'ion de terre rare complexé, l'un au moins desdits radicaux étant constitué par un système hétérocyclique azoté substitué ou non substitué dont l'un au moins des atomes d'azote porte un groupe oxy, et l'un des radicaux © et Ⓓ pouvant ne pas exister ;
- $X_1$ et $X_2$, identiques ou différents, représentent l'hydrogène ou une chaîne hydrocarbonée $(CH_2)_n$ éventuellement interrompue par un ou plusieurs hétéroatomes, dans laquelle n est un entier de 1 à 10, sous réserve que lorsque les radicaux Ⓐ et/ou Ⓑ représentent un système hétérocyclique azoté dont l'un au moins des atomes d'azote porte un groupe oxy, les radicaux © et/ou Ⓓ soient choisis parmi les biquinoléines, les bi-isoquinoléines, les bipyridines, les ter-pyridines, les coumarines, les bipyrazines, les bipyrimidines et les pyridines.

Dans un aspect préféré, les complexes macrocycliques de terres rares selon l'invention sont constitués d'au moins un sel de terre rare complexé par un composé macrocyclique de formule (I) ci-dessus dans laquelle l'un au moins des radicaux bivalents Ⓐ et Ⓑ comporte au moins un motif moléculaire ou est essentiellement constitué d'un motif moléculaire possédant une énergie de triplet supérieure à celle du niveau émissif de l'ion de terre rare complexé, et l'un au moins des radicaux © et Ⓓ est constitué par un système hétérocyclique azoté dont l'un au moins des atomes d'azote porte un groupe oxy.

D'autres complexes macrocyliques de terres rares avantageux sont ceux dans lesquels au moins l'une des conditions ci-dessous est réalisée :

- les radicaux bivalents Ⓐ et Ⓑ sont identiques ;

- les radicaux bivalents © et Ⓓ sont identiques ;
- $X_1$ et $X_2$ sont identiques ;

sous réserve que lorsque les radicaux Ⓐ et/ou Ⓑ représentent un système hétérocylique azote dont l'un au moins des atomes d'azote porte un groupe oxy, les radicaux © et/ou Ⓓ soient choisis parmi les biquinoléines, les bi-isoquinoléines, les bipyridines, les ter-pyridines, les coumarines, les bipyrazines, les bipyrimidines et les pyridines.

Les motifs moléculaires donneurs d'énergie triplet qui conviennent aux fins de l'invention doivent posséder une énergie triplet supérieure ou égale à celle des niveaux émissifs de l'ion de terre rare. Dans un aspect préféré, le niveau triplet dudit motif moléculaire est supérieur à 17 300 $cm^{-1}$.

Des motifs moléculaires particulièrement préférés aux fins de l'invention sont la phénantroline, l'anthracène, les bipyridines, les biquinoléines, notamment les bis-isoquinoléines, par exemple la 2,2'-bipyridine, les ter-pyridines, les coumarines, les bipyrazines, les bipyrimidines, l'azobenzène, l'azopyridine, les pyridines ou la 2,2'-bis-isoquinoléine, ou les motifs

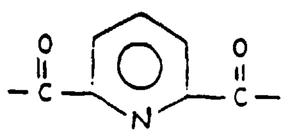 ; 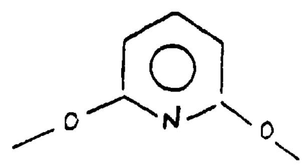 ;

ou

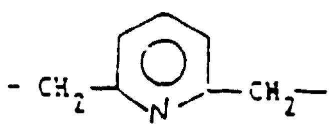

Dans un aspect avantageux, le système hétérocyclique azoté dont l'un au moins des atomes d'azote porte un groupe oxy est choisi parmi les motifs pyridine N-oxyde, bipyridine N-oxyde, bipyridine di-N-oxyde, bis-isoquinoléine N-oxyde, bis-isoquinoléine di-N-oxyde, bipyrazine N-oxyde, bipyrazine di-N-oxyde, bipyrimidine N-oxyde et bipyrimidine di-N-oxyde.

D'autres complexes macrocycliques préférés sont ceux dans lesquels le motif moléculaire possédant une énergie de triplet supérieure à celle du niveau émissif de l'ion de terre rare complexé et le système hétérocyclique azote dont l'un au moins des atomes d'azote porte un groupe oxy sont un seul et même motif.

En tant qu'ion de terre rare utilisable dans les complexes de l'invention, on peut utiliser en particulier des ions de terbium, europium, samarium et dysprosium. On utilisera de préférence le terbium ou l'europium.

Les complexes macrocycliques conviennent notamment à titre de marqueurs fluorescents de substances biologiques.

A cet effet, les complexes selon l'invention peuvent être substitués sur au moins l'un des radicaux Ⓐ, Ⓑ, © et Ⓓ du composé macrocyclique par un groupe -CO-NH-Y-Z dans lequel :

- Y est un groupe ou un bras d'espacement qui est constitué par un radical organique bivalent, choisi parmi les groupes alkylène linéaires ou ramifiés en $C_1$ à $C_{20}$, contenant éventuellement une ou plusieurs doubles liaisons et/ou étant éventuellement interrompus par un ou plusieurs hétéroatomes, tels que l'oxygène, l'azote, le soufre ou le phosphore, parmi les groupes cycloalkylène en $C_5$-$C_8$ ou parmi les groupes arylène en $C_6$ à $C_{14}$, lesdits groupes alkylène, cycloalkylène ou arylène étant éventuellement substitués par des groupes alkyle, aryle ou sulfonate.
- Z est un groupe fonctionnel susceptible de se lier de façon covalente avec une substance biologique.

Dans la présente description, on désigne par "groupe fonctionnel susceptible de se lier de façon covalente avec une substance biologique" tout groupe fonctionnel capable de se lier par liaison covalente, directement ou après activation, avec au moins l'une des fonctions naturellement présentes ou artificiellement introduites sur ladite substance biologique. De telles fonctions sont notamment les fonctions $NH_2$, COOH, SH ou OH. De tels groupes ainsi que les procédés d'activation sont décrits en détail par P. TIJSSEN dans "Practice and Theory of Enzyme immunossays" Elsevier 1985, document incorporé dans la présente description à titre de référence.

A titre d'exemples de groupes fonctionnels appropriés aux fins de l'invention, on peut citer notamment les groupes amino, thio, cyano, isocyano, isothiocyano, thiocyano, carboxyle, hydroxyle, maléimido, succinimido, marcapto, phénol, imidazole, aldéhyde, époxyde, halogénure, thionyle, sulfonyle, nitrobenzoyle, carbonyl, triazo, anhydride, halogénoacétate, hydrazino, acridine, etc.

Les groupes particulièrement préférés sont les groupes amino, thio et carboxy qui doivent être activés avant le couplage covalent avec la substance biologique ainsi que les groupes maléimido, succinimido et isothiocyanate, lesquels peuvent se lier directement avec la substance biologique.

Les complexes macrocycliques de l'invention sont préparés selon des procédés connus.

Les composés macrocycliques sont préparés selon les techniques décrites dans R. Ziessel et al, Helvetica Chimica Acta, 1990, 73, 1149 pour les macrocycles comprenant des motifs bipyridine. Les autres macrocycles peuvent être préparés selon les techniques décrites dans la série d'ouvrage Supramolecular Chemistry, Springer Verlag Chemie, F. Fögter Ed. On fait ensuite réagir l'unité macrocyclique de base avec un dérivé halogéné du système hétérocyclique azoté dans un solvant aprotique polaire, celui-ci ayant été préalablement oxydé, notamment par l'action d'un peracide tel que l'acide méta-chloroperbenzoïque.

Lorsque l'on souhaite préparer un complexe dans lequel les radicaux Ⓐ et/ou Ⓑ est constitué par un système hétérocyclique azoté dont l'un au moins des atomes d'azote porte un groupe oxy, on prépare le macrocycle de base que l'on oxyde de préférence lors de sa synthèse lorsqu'il se trouve sous forme d'un dérivé tosylé avec un peracide tel que l'acide méta-chloroperbenzoïque. Le composé macrocyclique tosylé est ensuite déprotégé et mis en réaction avec le dérivé halogéné des radicaux © et/ou Ⓓ.

La préparation du macrocycle bipyridine passant par un intermédiaire tosylé est notamment décrite dans J. Org. Chem, 1983, 48, 1848.

Les complexes macropolycycliques de terres rares selon l'invention peuvent être obtenus par les procédés classiques de préparation de complexes métalliques, qui consistent à faire réagir le composé complexant avec un composé donneur du cation à complexer.

Par exemple, les complexes macropolycycliques peuvent être obtenus par réaction d'un composé donneur de cation de terre rare avec le composé macropolycyclique ayant les caractéristiques définies ci-dessus, chaque composé étant avantageusement en solution, de préférence dans le même solvant ou dans des solvants compatibles inertes vis-à-vis de la complexation. En général, on utilise comme solvant l'acétonitrile ou le méthanol, en chauffant à reflux.

Dans un autre de ses aspects, l'invention concerne un procédé de réduction d'interférences dans un dosage par fluorescence utilisant comme traceurs les complexes macrocycliques précédemment décrits, notamment lorsque le milieu de mesure est un milieu biologique et en particulier un milieu sérique.

Dans un aspect ultérieur, l'invention concerne l'utilisation des complexes macrocycliques décrits ci-dessus pour réduire les interférences dans le milieu de mesure d'un procédé de détection et/ou de détermination par fluorescence d'un analyte dans un milieu susceptible de le contenir.

Lesdits complexes trouvent une application importante dans les dosages immunologiques par fluorescence, aussi bien dans les procédés de dosage dits par compétition, ou par excès, en phase homogène ou hétérogène, décrits dans l'art antérieur (Landon, Ann. Clin. Biochem, 1981, 18, 253 et E. SOINI et al, Clin. Chem. 1979, 25, 353).

Dans la présente description on définit par :

- "analyte" toute substance ou groupe de substances analogues à détecter et/ou déterminer ;
- "récepteur" toute substance capable de se fixer spécifiquement sur un site dudit analyte.

Dans un aspect préféré, le procédé de détection et/ou de détermination d'un analyte dans lequel on utilise les macrocycles de l'invention est un procédé homogène.

En particulier, on utilisera les macrocycles de l'invention dans un procédé de détection et/ou de détermination par fluorescence d'un analyte dans un milieu susceptible de le contenir consistant :

1) à ajouter audit milieu un premier réactif constitué d'au moins un récepteur dudit analyte,
2) à ajouter au second réactif choisi parmi l'analyte ou au moins l'un de ses récepteurs, l'un des deux réactifs étant couplé avec un composé fluorescent donner constitué par un complexe macrocyclique selon l'invention et l'autre réactif étant couplé avec un composé fluorescent accepteur et l'ordre d'ajout des réactifs pouvant être inversé,
3) faire incuber le milieu résultant soit après l'addition de chaque réactif, soit après l'addition des deux réactifs,
4) à exciter le milieu résultant à la longueur d'onde d'excitation du composé donneur,
5) à mesurer, à l'équilibre ou en cinétique, le signal émis par le composé fluorescent accepteur.

on peut également utiliser lesdits complexes dans un procédé de détection et/ou de détermination par fluorescence d'un analyte dans un milieu susceptible de le contenir à l'aide d'une méthode par excès consistant :

1) à ajouter audit milieu contenant l'analyte recherché, un premier réactif constitué par au moins un récepteur dudit analyte, couplé avec un composé fluorescent donneur constitué par un complexe macrocyclique selon l'invention,
2) à ajouter un second réactif constitué par un ou plusieurs autres récepteurs dudit analyte, ledit second réactif étant couplé avec un composé fluorescent accepteur,
3) à faire incuber ledit milieu après chaque addition de réactifs ou après l'addition des deux réactifs,
4) à exciter le milieu résultant à la longueur d'onde d'excitation du composé fluorescent donneur par une source de lumière,
5) à mesurer le signal émis par le composé fluorescent accepteur.

Dans la méthode par excès ci-dessus, on utilisera en particulier un seul récepteur de l'analyte qui peut être couplé soit avec le composé fluorescent donneur, soit avec le composé fluorescent accepteur.

Dans un aspect avantageux, on utilisera les complexes macrocycliques selon l'invention dans un procédé de détection et/ou de détermination par fluorescence d'un analyte dans un milieu susceptible de le contenir à l'aide d'une méthode par compétition consistant :

1) à ajouter audit milieu contenant l'analyte recherché, un premier réactif constitué par un récepteur dudit analyte, couplé avec un composé fluorescent donneur constitué par un complexe macrocyclique selon l'invention,
2) à ajouter un second réactif constitué de l'analyte couplé avec un composé fluorescent accepteur,
3) à faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent acepteur.

En particulier, on utilisera les complexes macrocycliques décrits précédemment dans un procédé de détection et/ou de détermination par fluorescence d'un analyte dans un milieu susceptible de le contenir à l'aide d'une méthode par compétition consistant :

1) à ajouter audit milieu contenant l'analyte recherché, un premier réactif constitué par un récepteur dudit analyte, ledit récepteur étant couplé avec un composé fluorescent accepteur,
2) à ajouter, à titre de second réactif, l'analyte étant couplé avec un composé fluorescent donneur constitué par un complexe macrocyclique selon l'invention,
3) à faire incuber ledit milieu soit après l'addition de chaque réactif, soit après l'addition des deux réactifs,
4) à exciter le milieu résultant à la longueur d'onde d'excitation du composé fluorescent donneur,
5) à mesurer le signal émis par le composé fluorescent accepteur.

Dans un aspect avantageux, le premier réactif et le second réactif utilisés dans les procédés de détection et/ou de détermination par fluorescence d'un analyte indiqués ci-dessus sont ajoutés simultanément au milieu contenant l'analyte recherché.

De préférence, on utilisera en tant que composé fluorescent donneur un complexe macrocyclique dans lequel l'ion de terre rare est l'europium et comme composé fluorescent accepteur un composé choisi parmi l'allophycocyanine, l'allophycocyanine B, la C phycocyanine ou la R phycocyanine.

On peut également utiliser en tant que composé fluorescent donneur un complexe macrocyclique de terbium et comme composé fluorescent accepteur un composé choisi parmi les rhodamines, la thionine, la R phycocyanine, la phycoérythrocyanine, la C phycoérythrine, la B phycoérythrine ou la R phycoérythrine.

L'invention sera mieux comprise à l'aide des exemples ci-après qui ne présentent aucun caractère limitatif.

Exemple 1

Préparation du composé macrocyclique de formule (3) :

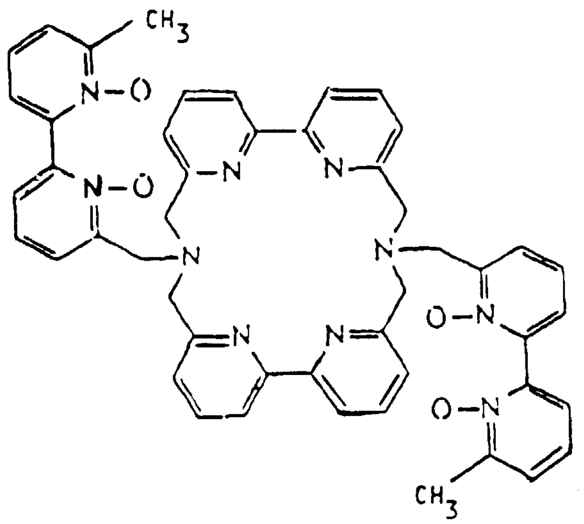

(3)

Ce composé est préparé selon le schéma ci-dessous :

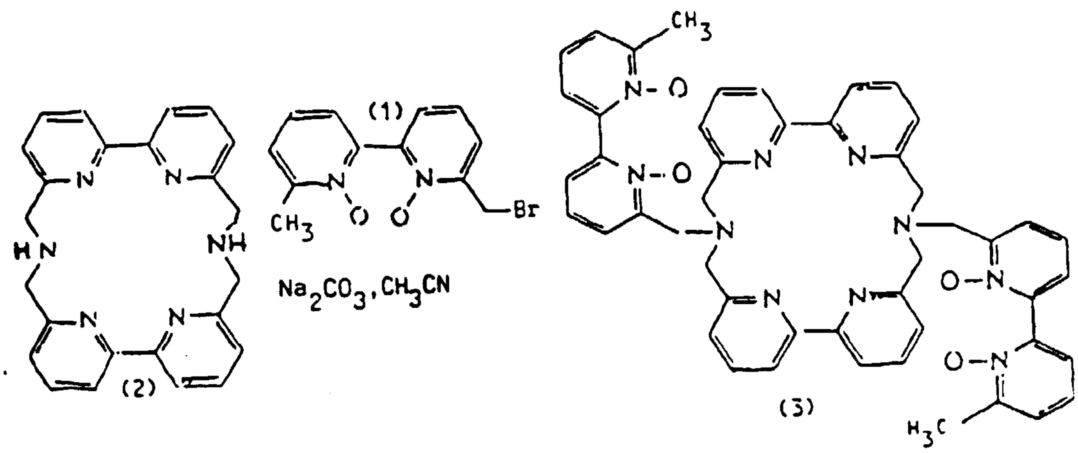


a) Préparation du composé (1)

Un mélange de 6,6'-diméthyl-2,2'-bipyridine (2,76 g, 15 mmol) et de N-bromosuccinimide (5,10 g, 28,6 mmol) est chauffé au reflux dans 150 ml de chloroforme pendant 30 min. On ajoute ensuite 30 mg de peroxyde de benzoyle. Le mélange est alors chauffé au reflux pendant 2 h, puis filtré pour séparer le succinimide.

La solution est refroidie à 0°C et le solide qui se dépose est filtré et lavé au méthanol. On récupère 1,65 g de 6,6'-bis(bromométhyl)-2,2'-bipyridine sous forme d'un cristal solide blanc. Le filtrat obtenu précédemment à partir de la solution dans le chloroforme est concentré et chromatographié sur colonne de silice (éluant : $CH_2Cl_2$/MeOH 98:2).

On obtient 1,38 g de 6,6'-bis(bromométhyl)-2,2'-bipyridine, 0,55 g de 6-méthyl-6'-bromométhyl-2,2'-bipyridine (point de fusion : 88°C) et 0,9 g de 6,6'-bis(dibromométhyl)-2,2'-bipyridine.

On solubilise 0,5 g (1,9 mmol) de 6-méthyl-6'-bromométhyl-2,2'-bipyridine dans 100 ml de chloroforme passé préalablement sur alumine (Basic-activity I, Merck, USA). On ajoute goutte à goutte 1,2 g d'acide métachloroperbenzoïque (55 % $H_2O$), soit 2 équivalents d'acide pour un équivalent du dérivé bromé, en solution dans 50 ml de chloroforme. Après 4 h, on ajoute goutte à goutte 2 autres équivalents d'acide métachloroperbenzoïque. Après agitation pendant une nuit à température ambiante, on évapore à sec et on sèche sur vide de pompe à palette. On lave 5 fois à l'éther et on obtient 400 mg de produit attendu sous forme d'une poudre jaune pâle (rendement : 71 %). Point de

fusion : 125°C.

b) Préparation du composé (3)

A un mélange contenant 0,45 g (1,14 mmol) de composé (2) (macrocycle bipyridine, décrit dans J. Org. Chem., 1983, 48, 4848) et 3 g (17 mmol) de $Na_2CO_3$ dans 300 ml de MeCN fraîchement distillé et sous atmosphère d'azote, sous agitation et à reflux, on ajoute goutte goutte 1,1 g (3,70 mmol) du composé (1) en solution dans 200 ml de $CH_3CN$.

On maintient sous agitation et à reflux pendant 24 h. Le mélange est filtré à chaud et le filtrat est concentré sous vide à température ambiante. Le produit brut est mis en solution dans $CHCl_3$ (200 ml) et lavé 3 fois à l'eau, séché sur $MgSO_4$ et évaporé. Après chromatographie sur alumine (éluant : $CH_2Cl_2$/MeOH 9:1) on botient 400 mg de produit attendu sous forme d'une poudre blanche (rendement : 42 %). Point de fusion : 235°C (décomposition).

Spectre $^1$H-RMN ($CDCl_3$) :
2,59 ($2CH_3$) ; 4,21 ($4CH_2$) ; 4,55 ($2CH_2$) ;
7,09 (d, J=7,3, 4H) ; 7,31-7,47 (m, 14H) ;
7,74 (d, J=7,5, 4H) ; 8,22-8,27 (m, 2H) ;

Spectre $^{13}$C-RMN ($CD_3OD$) ;
18,1 ($2CH_3$) ; 58,2 ($2CH_2$) ; 62,0 ($4CH_2$) ;
121,2 ; 124,9 ; 125,5 ; 127,7 ; 128,1 ; 128,5 ;130,8 ; 139,3 : (24H)
145,3 ; 145,4 ; 148,8 ; 150,4 ; 156,0 ; 159,9 : (16C)

| Analyse élémentaire : | | | |
|---|---|---|---|
| - calculé pour $C_{48}H_{44}O_5N_{10},H_2O$ (840,91) | | | |
| | C:68,55 | H:5,27 | N:16,66 |
| | C:68,55 | H:5,27 | N:16,66 |
| - trouvé | C:68,34 | H:5,09 | N:16,38 |

Exemple 2

Préparation d'un complexe macrocyclique d'europium (macrocycle : composé (3) de l'exemple 1)

17 mg de $EuCL_3,6H_2O$ ($4,6.10^{-5}$ mol) sont ajoutés sous agitation à une solution de 37 mg de composé (3) ($4,5.10^{-5}$ mol) dans 15 ml de méthanol à température ambiante. L'agitation est maintenue pendant 48 h sous atmosphère d'azote. On ajoute ensuite 30 ml de $Et_2O$, ce qui provoque l'apparition d'un précipité blanc que l'on isole par centrifugation. On récupère ainsi 50 mg (99 %) de produit attendu sous forme de poudre blanche. Point de fusion : 190°C (décomposition).

Spectre de masse FAB-MS (NBA) :
1045,0 ($[M+Eu+2Cl]^+$) ; 1010,1($[M+Eu+Cl]^+$) ; 994,1($[M-O+Eu+Cl]^+$)

| Analyse élémentaire : | | | |
|---|---|---|---|
| - calculé pour $C_{48}H_{42}O_4N_{10},EUCl_36H_2O$ (1189,30) | | | |
| | C:48,47 ; | H:4,57 ; | N:11,78 |
| - trouvé | C:48,71 ; | H:4,94 ; | N:10,89 |

Exemple 3

Préparation d'un complexe macrocyclique de terbium (macrocycle : composé (3) de l'exemple 1)

- 12 g de $TbCl_3,6H_2O$ ($3,2.10^{-5}$ mol) sont ajoutés sous agitation à une solution de 25 mg de composé (3) ($3,04.10^{-5}$ mol) dans 15 ml de méthanol à température ambiante. L'agitation est maintenue 48 h sous atmosphère d'azote. on ajoute ensuite 30 ml de $Et_2O$, ce qui provoque l'apparition d'un précipité blanc isolé par centrifugation. On récupère ainsi 33 mg (94 %) de produit attendu sous forme d'une poudre jaune. Point de fusion : 190°C (décomposition).

Spectre de masse FAB-MS (NRA) :
1050,9 ($[M+Tb+2Cl^-]^+$) : 1014,9 ($[M+Tb+Cl]^+$)

| Analyse élémentaire : | | | |
|---|---|---|---|
| - calculé pour $C_{48}H_{42}O_4N_{10},TbCl_3,H_2O$ (1106,19) : | | | |
| | C:52,11 ; | H:4,01 ; | N:12,66 |
| - trouvé | C:51,83 ; | H:4,22 : | N:12,28 |

Exemple 4

Préparation des composés macrocycliques de formules (5) et (6)

Ces composés sont préparés à partir du composé (2) de l'exemple 1 selon le schéma ci-dessous.

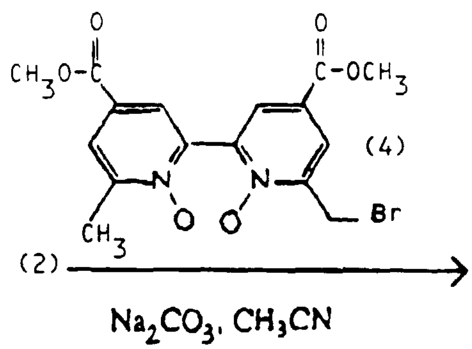

(4)

(2)

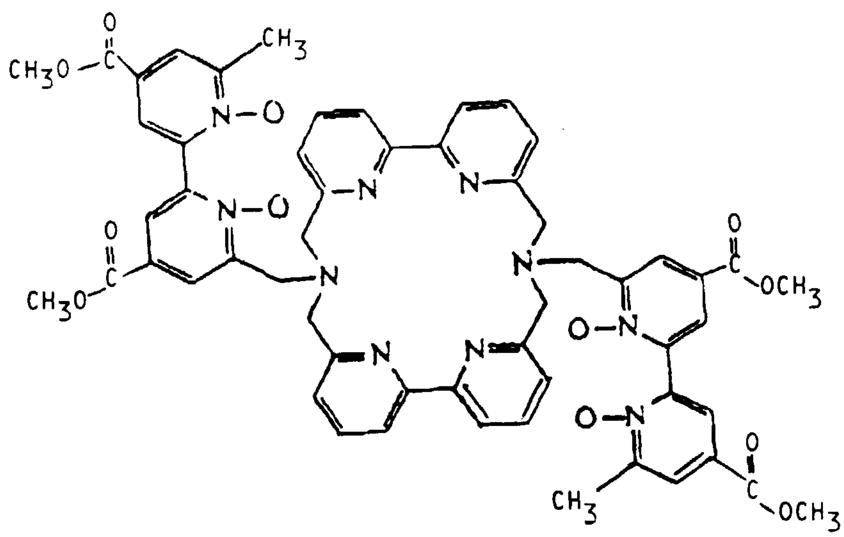

(5)

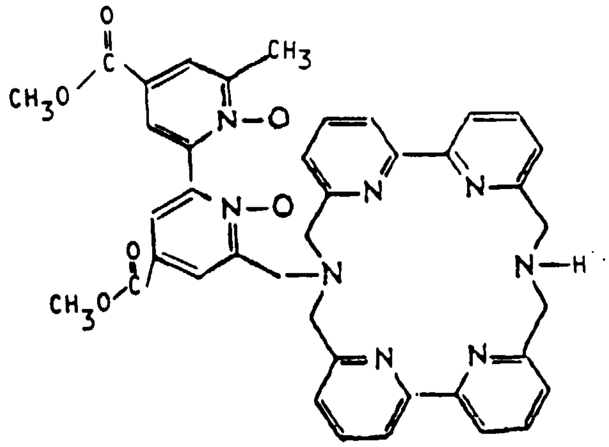

(6)

a. Préparation du composé (4)

Un mélange de 6,6'-diméthyl-2,2'-bipyridine-4,4'-dicarboxylate de diméthyle (4,4 g) et de N-bromosuccinimide (9,76 g) est chauffé au reflux pendant 30 min dans 240 ml de tétrachlorure de carbone.

On ajoute ensuite 0,54 g de 2,2'-azobis(2-méthylproprionitrile). Le mélange est chauffé au reflux pendant 4 h.

La solution est ensuite filtrée à chaud et refroidie pendant 12 h à 4°C.

Le précipité obtenu est évaporé et chromatographié sur colonne de silice (éluant : $CH_2Cl_2$/hexane 50/50).

On obtient 2,06 g de 6-méthyl-6'-bromométhyl-2,2'-bipyridine-4,4'-dicarboxylate de diméthyle.

On solubilise 2,06 g (5,4 mmol) de diméthyl-6-méthyl-6'-bromométhyl-2,2'-bipyridine-4,4'-dicarboxylate dans 200 ml de chloroforme. On ajoute goutte à goutte 3,35 g (0,1 mM) d'acide métachloroperbenzoïque en solution dans 40 ml de chloroforme. Après 12 h, on ajoute goutte à goutte une solution chloroformique contenant 6,7 g d'acide méta-chloroperbenzoïque.

Au bout de 24 h, on évapore à sec et on sèche sur vide de pompe à palette. On lave 4 fois par 50 ml d'éther éthylique, et on purifie sur silice (éluant : $CHCl_3$/cyclohexane 90/10). On obtient 1,05 g de produit attendu (rendement 47 %).

b. Préparation des composés (5) et (6)

On porte à reflux pendant 30 min un mélange contenant 48 mg (0,12 mM) de composé (2) et 0,13 g (1,2 mM) de $Na_2CO_3$ dans 150 ml de $CH_3CN$.

On ajoute ensuite goutte à goutte un mélange contenant 100 mg (0,24 mM) de composé (1) en solution dans 50 ml de $CH_3CN$.

On maintient sous agitation et à reflux pendant 24 h. Le mélange est filtré à chaud et le filtrat concentré sous vide.

Exemple 5

Préparation de complexes macrocycliques d'europium dont les macrocycles sont les composés (5) et (6) de l'exemple 4

Au produit brut obtenu à l'exemple 4, on ajoute 302 mg de $EuCl_3$, $6H_2O$ (0,82 mM) dans 60 ml de méthanol anhydre. On porte le mélange à reflux pendant 2 h.

La solution est ensuite refroidie et évaporée. Le brut de réaction est ensuite purifié sur colonne phase inverse dans $CH_3CN$/ $H_2O$/TFA.

On obtient :

- 18 mg de complexe macrocyclique (6)-spectre de masse FAB-MS(NBA) :

  1101 [M + EU + $2CF_3COO^-$]
  988 [M + Eu + $CF_3COO^-$]

- 29 mg de complexe macrocyclique (5)-spectre de masse FAB-MS(NBA) :
  1431 [M + Eu + $2CF_3COO^-$]

Exemple 6

Préparation d'un composé macrocyclique de formule (7)

Ce composé est préparé à partir des composés (1) et (2) de l'exemple 1 et du composé (4) de l'exemple 4 selon le schéma ci-dessous.

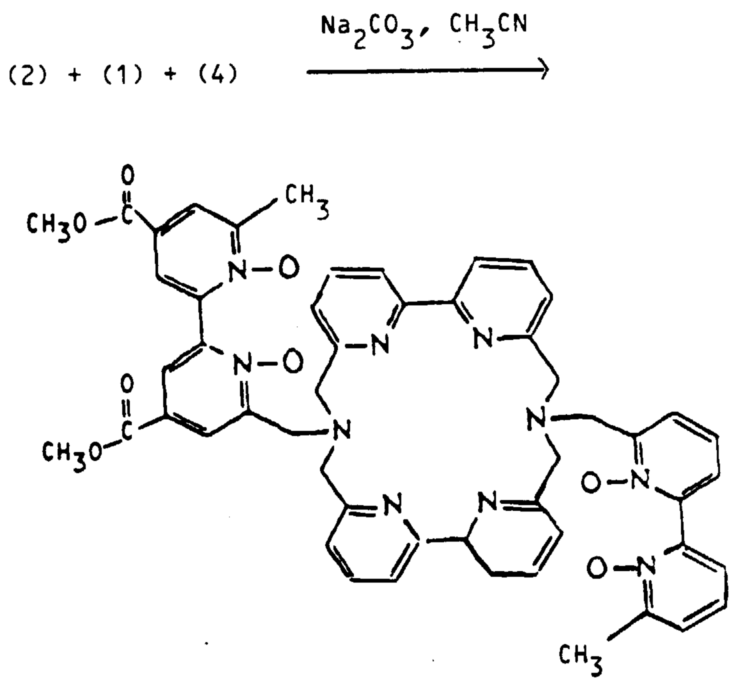


(7)

A un mélange de 0,45 g (1,14 mM) de composé (2) et 3 g (17 mM) de $Na_2CO_3$ dans 150 ml de $CH_3CN$, et 125 ml de $CH_2Cl_2$, on ajoute sous reflux et au goutte à goutte 0,55 g (1,8 mM) de composé (1) en solution dans 100 ml de $CH_3CN$.

On maintient sous agitation et reflux pendant 12 h. Le mélange est filtré à chaud et concentré sous vide. Le produit brut est purifié sur colonne d'alumine. On obtient 207 mg du produit monosubstitué.

A un mélange de 0,20 g de produit monosubstitué (0,23 mM) et de 0,6 g (3,4 mM) de $Na_2CO_3$ dans 60 ml de $CH_3CN$, on ajoute sous reflux et au goutte à goutte 0,1 g de composé (4) (0,24 mM) en solution dans 20 ml de $CH_3CN$.

La solution est maintenue sous agitation et reflux pendant 24 h. Le mélange est filtré à chaud et concentré sous vide. Le produit est lavé à l'eau.

On obtient 57 mg de composé (7) (0,05 mM).

Exemple 7

Préparation d'un complexe macrocyclique d'europium dont le macrocycle est le composé (7) de l'exemple 6

Ce complexe est préparé comme décrit plus haut à l'exemple 5.

Spectre de masse FAB-MS(NBA) :

1161 [M + Eu + 2Cl]
1126 [M + Eu + Cl]

Exemple 8

Préparation d'un complexe macrocyclique d'europium dont le macrocycle est le composé de formule (8)

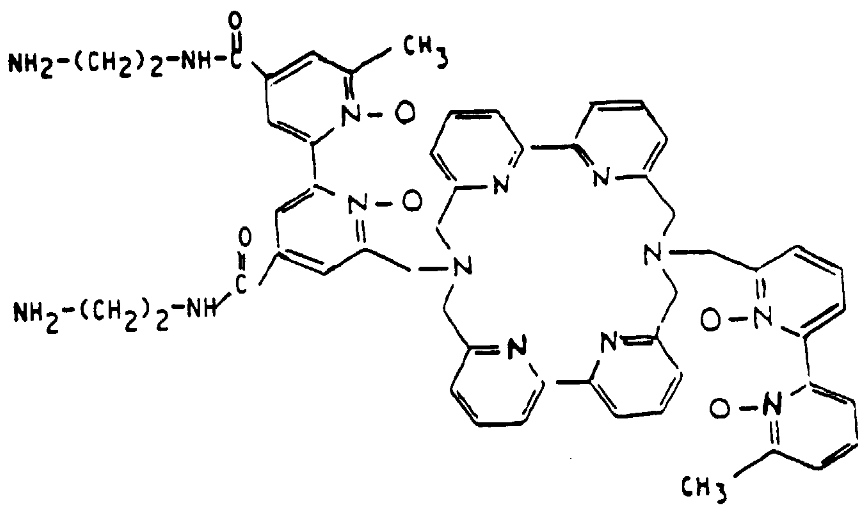

(8)

Ce composé est obtenu à partir du macrocycle de l'exemple 7 à l'aide d'une réaction d'aminolyse telle que décrite dans le brevet EP 0 321 353 (voir notamment exemple 3, étape B). Brièvemement, le macrocycle est soumis à une aminolyse en l'ajoutant par portion à 4 ml d'éthylènediamine distillée sur KOH préalablement chauffée à 90°C. Le mélange réactionnel obtenu est encore agité pendant 1 h puis refroidi à la température ambiante. L'éthylènediamine en excès est ensuite éliminée sous vide et on obtient une huile. Cette dernière est reprise dans 2 ml d'un mélange de toluène/$CH_3OH$ (2/1) qui est à son tour éliminé sous vide. On obtient alors une poudre beige. On dose par colorimétrie 1,8 $NH_2$ par mole de chélate.

Exemple 9

Mise en évidence de la stabilité et de la nom-sensibilité ou "quenching" (extinction) des complexes macrocycliques d'europium des exemples 2, 5, 7 et 8.

Les déterminations ci-dessous ont été effectuées avec un spectromètre PERKIN-ELMER LS 5.

La mesure de la durée de vie des complexes testés a été effectuée de la manière suivante : on a enregistré le spectre d'émission du pic situé entre 600 et 650 nm, la solution étant excitée dans l'un des pics d'absorption. On a opéré en mode "phosphorescence", la valeur de la fenêtre a été fixée à 1 ms = tg. Les enregistrements ont été réalisés pour plusieurs valeurs de $t_d$ (délai), soit 0,1 ; 0,2 ; 0,3 ; 0,4 et 0,5 ms. On a mesuré l'intensité du pic $I_t$ et déterminé T par la formule :

$$I_t = I_o\, e^{\frac{t}{\tau}}$$

$$\tau = \frac{0{,}434\, t}{\log I_o - \log I_t}$$

Les composés ont été testés dans l'eau et dans un tampon phosphate 100 mM, pH 7,2 une concentration en complexe d'environ $10^{-6}$ M/L.

Les complexes comportent les composés macrocycliques ci-après ont été testés en comparaison avec les complexes d'europium des exemples 2, 5, 7 et 8.

- Composé A :

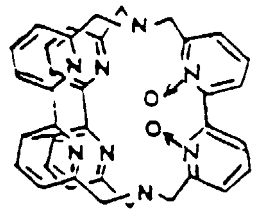

- Composé B :

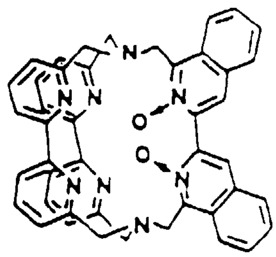

- Composé C :

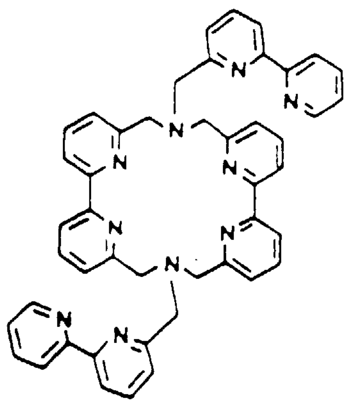

- Composé D :

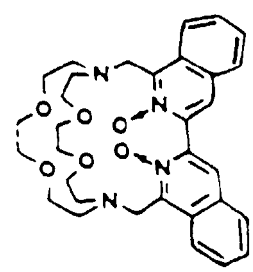

- Composé E :

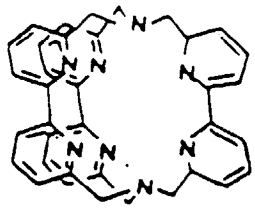

La préparation des composés A et B est décrite dans J. M. Lehn et al, Helvetica Chimica Acta, 1991, 74, 572 ; celle du composé C est décrite dans R. Ziessel et al, Helvetica Chimica Acta, 1990, 73, 1149; celle du composé D dans J. M. Lehn et al, Helvetica Chimica Acta, 1990, 73, 106 et celle du composé E dans le brevet EP 0 180 492.

Les résultats sont donnés dans le tableau 1 ci-après.

Les mesures dans le sérum ont été effectuées en sérum humain dilué au 1/3 avec du tampon phosphate 100 mM, pH 7,2.

Les durées de vie (T) sont exprimées en ms.

Tableau 1

| Complexe testé | $\tau\ H_2O$ | $\tau$ tampon $PO_4$ | $\tau$ sérum | Stabilité tampon $PO_4$ | Quenching sérum | |
|---|---|---|---|---|---|---|
| | | | | | $\left(1-\frac{\tau\ \text{sérum}}{\tau\ PO_4}\right)\%$ | $\left(1-\frac{\tau\ \text{sérum}}{\tau\ H_2O}\right)\%$ |
| Exemple 2 macrocycle (3) | 0,48 | 0,66 | 0,62 | Stable | 6 % | exaltation + 30 % |
| Exemple 5 macrocycle (5) | 0,15 | - | 0,19 | Stable | - | exaltation + 27 % |
| Exemple 5 macrocycle (6) | 0,17 | - | 0,19 | Stable | - | exaltation + 12 % |
| Exemple 7 macrocyle (7) | 0,38 | - | 0,44 | Stable | - | exaltation + 16 % |
| Exemple 8 macrocyle (8) | 0,38 | 0,44 | 0,53 | Stable | exaltation + 20 % | exaltation + 39 % |
| A | 0,46 | 0,84 | 0,2 | Stable | 75 % | 56 % |
| B | 0,39 | 0,49 | 0,14 | Stable | 71 % | 64 % |
| C | 1,5 | 1,2 | 0,7 Modification du spectre d'émission | Perd la moitié du signal en 1h30 et formation d'un précipité | 40 % Modification du spectre d'émission | 53 % |
| D | 0,38 | 0,63 | 0,2 | Stable | 68 % | - |
| E | 0,34 | 0,52 | 0,15 | Stable | 71 % | 47 % |

Les résultats montrent que bien que la plupart des composés testés soient stables dans le tampon phosphate (stabilité évaluée par la permanence de la hauteur du pic), seul les complexes selon l'invention sont significativement

peu sensibles au phénomène de quenching, ou présentent même une exaltation de la fluorescence dans le sérum par rapport à la fluorescence émise dans l'eau.

## Revendications

1. Complexes macrocycliques de terres rares, caractérisés en ce qu'ils sont constitués d'au moins un sel de terre rare complexé par un composé macrocyclique de formule (I)

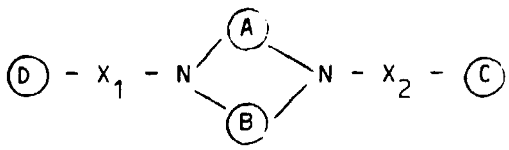

(I)

dans laquelle

- les radicaux bivalents Ⓐ, Ⓑ, © et Ⓓ, identiques ou différents, représentent des chaines hydrocarbonées contenant éventuellement un ou plusieurs hétéroatomes, l'un au moins desdits radicaux comportant au moins un motif moléculaire ou étant essentiellement constitué par un motif moléculaire possédant une énergie de triplet supérieure à celle du niveau émissif de l'ion de terre rare complexé, l'un au moins desdits radicaux étant constitué par un système hétérocyclique azoté substitué ou non substitué dont l'un au moins des atomes d'azote porte un groupe oxy, et l'un des radicaux © ou Ⓓ pouvant ne pas exister ;
- $X_1$ et $X_2$, identiques ou différents, représentent l'hydrogène ou une chaine hydrocarbonée $(CH_2)_n$ éventuellement interrompue par 1 ou plusieurs hétéroatomes, dans laquelle n est un entier de 1 à 10, sous réserve que lorsque les radicaux Ⓐ et/ou Ⓑ représentent un système hétérocyclique azoté dont l'un au moins des atomes d'azote porte un groupe oxy, les radicaux © et/ou Ⓓ soient choisis parmi les biquinoléines, les bi-isoquinoléines, les bipyridines, les ter-pyridines, les coumarines, les bipyrazines, les bipyrimidines et les pyridines.

2. Complexes selon la revendication 1, caractérisés en ce que l'un au moins des radicaux bivalents Ⓐ et Ⓑ comporte au moins un motif moléculaire ou est essentiellement constitué d'un motif moléculaire possédant une énergie de triplet supérieure à celle du niveau émissif de l'ion de terre rare complexé, et l'un au moins des radicaux © et Ⓓ est constitué par un système hétérocyclique azoté dont l'un au moins des atomes d'azote porte un groupe oxy.

3. Complexes selon l'une des revendications 1 ou 2, caractérisés en ce que les radicaux Ⓐ et Ⓑ sont identiques.

4. Complexes selon l'une des revendications ou 2, caractérisés en ce que les radicaux © et Ⓓ sont identiques.

5. Complexes selon l'une quelconque des revendications 1 à 4, caractérisés en ce que $X_1$ et $X_2$ sont identiques.

6. Complexes selon l'une quelconque des revendications 1 à 5, caractérisés en ce que l'énergie de triplet du motif moléculaire est supérieure à 17 300 $cm^{-1}$.

7. Complexes selon l'une quelconque des revendications 1 à 6, caractérisés en ce que le motif moléculaire est choisi parmi la phénanthroline, l'anthracène, les bipyridines, les biquinoléines, les ter-pyridines, les coumarines, les bipyrazines, les bipyrimidines, l'azobenzène, l'azopyridine, les pyridines ou la 2,2'-biisoquinoléine, ou les motifs :

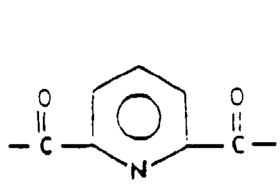

;

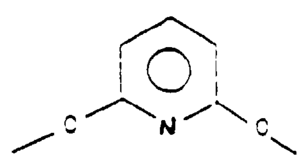

;

ou

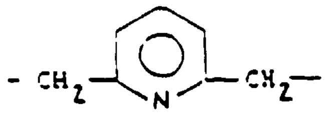

8. Complexes selon l'une quelconque des revendications 1 à 7, caractérisés en ce que le système hétérocyclique azoté est choisi parmi les motifs pyridine N-oxyde, bipyridine N-oxyde, bipyridine di-N-oxyde, bis-isoquinoléine N-oxyde et bis-isoquinoléine di-N-oxyde.

9. Complexes selon l'une quelconque des revendications 1 à 9, caractérisés en ce que le motif moléculaire possédant une énergie de triplet supérieure à celle du niveau émissif de l'ion de terre rare complexé et le système hétérocyclique azoté dont l'un au moins des atomes d'azote porte un groupe oxy sont un seul et même motif.

10. Complexes selon l'une quelconque des revendications 1 à 9, caractérisés en ce que l'ion de terre rare est choisi parmi l'europium, le terbium, le samarium et le dysprosium.

11. Complexes selon l'une quelconque des revendications 1 à 10, caractérisés en ce que au moins l'un des radicaux Ⓐ, Ⓑ, © et Ⓓ est substitué par un groupe -CO-NH-Y-Z dans lequel

- Y est un groupe ou un bras d'espacement qui est constitué par un radical organique bivalent, choisi parmi les groupes alkylène linéaires ou ramifiés en $C_1$ à $C_{20}$, contenant éventuellement une ou plusieurs doubles liaisons et/ou étant éventuellement interrompus par un ou plusieurs hétéroatomes, tels que l'oxygène, l'azote, le soufre ou le phosphore, parmi les groupes cycloalkylène en $C_5$-$C_8$ ou parmi les groupes arylénes en $C_6$-$C_{14}$, lesdits groupes alkylène, cycloalkylène ou arylène étant éventuellement substitués par des groupe alkyle, aryle ou sulfonate.
- Z est un groupe fonctionnel susceptible de se lier de façon covalente avec une substance biologique.

12. Complexes selon l'une quelconque des revendications 1 à 10, caractérisés ence que le composé macrocyclique est le composé de formule

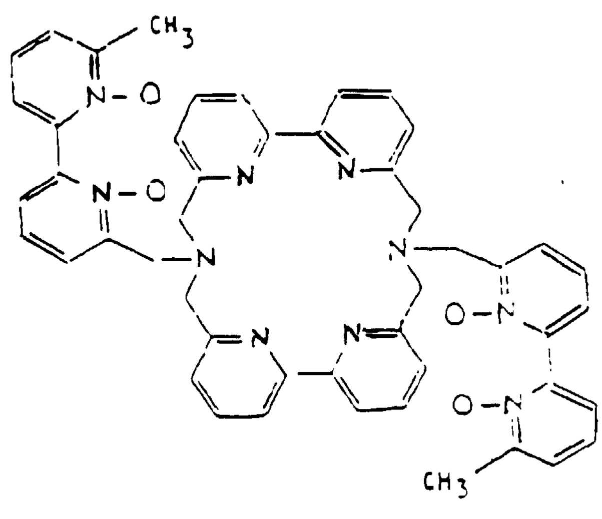

13. Procédé de réduction d'interférences dans un dosage par fluorescence d'un analyte, caractérisé en ce qu'on utilise comme traceur un complexe macrocyclique selon l'une quelconque des revencations 1 à 12.

14. Procédé selon la revendication 13, caractérisé en ce que le milieu de mesure est un milieu biologique.

15. Procédé selon la revendication 14, caractérisé en ce que le milieu de mesure est un milieu sérique.

16. Utilisation d'un complexe macrocyclique de terre rare selon l'une quelconque des revendications 1 à 12, pour réduire les interférences dans le milieu de mesure d'un procédé de détection et/ou de détermination par fluorescence d'un analyte dans un milieu susceptible de le contenir.

**17.** Utilisation selon la revendication 16, dans un procédé homogène de détection et/ou de détermination par fluorescence d'un analyte dans un milieu susceptible de le contenir.

**18.** Utilisation selon l'une des revendications 16 ou 17, dans un procédé de détection et/ou de détermination par fluorescence d'un analyte dans un milieu susceptible de le contenir consistant :

1) à ajouter audit milieu un premier réactif constitué d'au moins un récepteur dudit analyte,
2) à ajouter un second réactif choisi parmi l'analyte ou au moins l'un de ses récepteurs, l'un des deux réactifs étant couplé avec un composé fluorescent donneur constitué par un complexe selon l'une quelconque des revendications 1 à 12 et l'autre réactif étant couplé avec un composé fluorescent accepteur, l'ordre d'ajout des réactifs pouvant être inversé,
3) à faire incuber le milieu résultant soit après l'addition de chaque réactif, soit après l'addition des deux réactifs,
4) à exciter le milieu résultant à la longueur d'onde d'excitation du composé donneur,
5) à mesurer, à l'équilibre ou en cinétique, le signal émis par le composé fluorescent accepteur.

**19.** Utilisation selon la revendication 18, dans un procédé de détection et/ou de détermination car fluorescence d'un analyte dans un milieu susceptible de le contenir à l'aide d'une méthode par excès consistant :

1) à ajouter, audit milieu contenant l'analyte recherché, un premier réactif constitué par au moins un récepteur dudit analyte, couplé avec un composé fluorescent donneur constitué par un complexe selon l'une quelconque des revendications 1 à 12,
2) à ajouter un second réactif constitué par un ou plusieurs autres récepteurs dudit analyte, ledit second réactif étant couplé avec un composé fluorescent accepteur,
3) à faire incuber ledit milieu après chaque addition de réactifs ou après l'addition des deux réactifs,
4) à exciter le milieu résultant à la longueur d'onde d'excitation du composé fluorescent donneur par une source de lumière,
5) à mesurer le signal émis par le composé fluorescent accepteur.

**20.** Utilisation selon la revendication 18, dans un procédé de détection et/ou de détermination par fluorescence d'un analyte dans un milieu susceptible de le contenir à l'aide d'une méthode par compétition consistant :

1) à ajouter, audit milieu contenant l'analyte recherché, un premier réactif constitué par un récepteur dudit analyte, couplé avec un composé fluorescent donneur constitué par un complexe selon l'une quelconque des revendications 1 à 9,
2) à ajouter un second réactif constitué de l'analyte couplé avec un composé fluorescent accepteur,
3) a faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent accepteur.

**21.** Utilisation selon la revendication 18, dans un procédé de détection et/ou de détermination par fluorescence d'un analyte dans un milieu susceptible de le contenir à l'aide d'une méthode par compétition consistant :

1) à ajouter, audit milieu contenant l'analyte recherché, un premier réactif constitué par un récepteur dudit analyte, ledit récepteur étant couplé avec un composé fluorescent accepteur,
2) à ajouter, à titre de second réactif, l'analyte étant couplé avec un composé fluorescent donneur constitué par un complexe
3) selon l'une quelconque des revendications 1 à 9, à faire incuber ledit milieu soit après l'addition de chaque réactif, soit après l'addition des deux réactifs,
4) à exciter le milieu résultant à la longueur d'onde d'excitation du composé fluorescent donneur,
5) à mesurer le signal émis par le composé fluorescent accepteur.

**22.** Utilisation selon l'une quelconque des revendications 18 à 21, caractérisée en ce que le premier réactif et le second réactif sont ajoutés simultanément au milieu contenant l'analyte recherché.

**23.** Utilisation selon l'une des revendications 18 ou 19, caractérisée en ce qu'on utilise un seul récepteur de l'analyte, qui est couplé soit avec le composé fluorescent donneur, soit avec le composé fluorescent accepteur.

**24.** Utilisation selon l'une quelconque des revendications 18 à 23, caractérisée en ce que l'ion de terre rare du complexe macrocyclique utilisé comme composé fluorescent donneur est l'europium, et en ce que le composé fluorescent accepteur est choisi parmi l'allophycocyanine, l'allophycocyanine B, la C phycocyanine ou la R phycocyanine.

**25.** Utilisation selon l'une quelconque des revendications 18 à 23, caractérisée en ce que l'ion de terre rare du complexe macrocyclique utilisé comme composé fluorescent donneur est le terbium et en ce que le composé fluorescent accepteur est choisi parmi les rhodamines, la thionine, la R phycocyanine, la phycoérythrocyanine, la C phycoérythrine, la B phycoérythrine ou la R phycoérythrine.

## Patentansprüche

**1.** Makrocyclische Komplexe von Seltenerdmetallen, dadurch gekennzeichnet, daß
sie aus mindestens einem Seltenerdmetallsalz bestehen, das durch eine makrocyclische Verbindung der Formel (I)

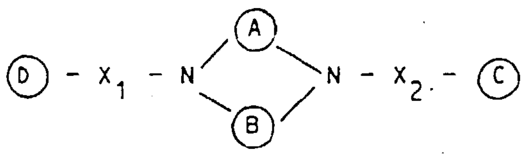

(I)

komplexiert ist, worin bedeuten:

- die zweiwertigen Gruppen Ⓐ, Ⓑ, © und Ⓓ, die identisch oder voneinander verschieden sind, Kohlenwasserstoffketten, die ggf. ein oder mehrere Heteroatome enthalten, wobei mindestens eine der Gruppen mindestens eine molekulare Einheit enthält oder im wesentlichen aus einer molekularen Einheit besteht, die eine Triplett-Energie über der Triplett-Energie des Emissionsniveaus des komplexierten Seltenerdmetallions aufweist, mindestens eine der Gruppen aus einem ggf. substituierten Stickstoffheterocyclus besteht, wobei mindestens eines der Stickstoffatome eine Oxygruppe trägt, und wobei eine der Gruppen © oder Ⓓ nicht vorhanden sein kann;
- $X_1$ und $X_2$, die identisch oder voneinander verschieden sind, Wasserstoff oder eine Kohlenwasserstoffkette $(CH_2)_n$, die ggf. durch ein oder mehrere Heteroatome unterbrochen ist, wobei n eine ganze Zahl im Bereich von 1 bis 10 ist,
mit der Maßgabe, daß die Gruppen © und/oder Ⓓ unter den Bichinolinen, Biisochinolinen, Bipyridinen, Terpyridinen, Cumarinen, Bipyrazinen, Bipyrimidinen und Pyridinen ausgewählt sind, wenn die Gruppen Ⓐ und/oder Ⓑ einen Stickstoffheterocyclus bedeuten, in dem mindestens eines der Stickstoffatome eine Oxygruppe trägt.

**2.** Komplexe nach Anspruch 1, dadurch gekennzeichnet, daß mindestens eine der zweiwertigen Gruppen Ⓐ und Ⓑ, mindestens eine molekulare Einheit enthält oder im wesentlichen aus einer molekularen Einheit gebildet ist, die eine Triplett-Energie über der Triplett-Energie des Emissionsniveaus des komplexierten Seltenerdmetallions aufweist, und mindestens eine der Gruppen © und Ⓓ aus einem Stickstoffheterocyclus besteht, in dem mindestens eines der Stickstoffatome eine Oxygruppe trägt.

**3.** Komplexe nach einem der Ansprüche 1 oder 2, dadurch gekennzeichnet, daß die Gruppen Ⓐ und Ⓑ identisch sind.

**4.** Komplexe nach einem der Ansprüche 1 oder 2, dadurch gekennzeichnet, daß die Gruppen © und Ⓓ identisch sind.

**5.** Komplexe nach einem der Ansprüche 1 bis 4, dadurch gekennzeichnet, daß $X_1$ und $X_2$ identisch sind.

**6.** Komplexe nach einem der Ansprüche 1 bis 5, dadurch gekennzeichnet, daß die Triplett-Energie der molekularen Einheit über 17300 $cm^{-1}$ liegt.

**7.** Komplexe nach einem der Ansprüche 1 bis 6, dadurch gekennzeichnet, daß die molekulare Einheit ausgewählt ist unter Phenanthrolin, Anthracen, Bipyridinen, Bichinolinen, Terpyridinen, Cumarinen, Bipyrazinen, Bipyrimidinen, Azobenzol, Azopyridin, Pyridinen oder 2,2'-Biisochinolin oder den Einheiten

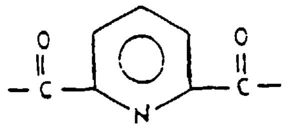 ; 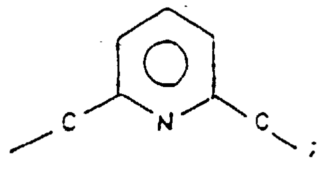

oder

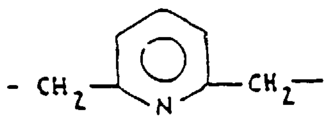

8. Komplexe nach einem der Ansprüche 1 bis 7, dadurch gekennzeichnet, daß der Stickstoffheterocyclus unter den Einheiten N-Oxypyridin, N-Oxybipyridin, Di-(N-Oxy)-bipyridin, N-Oxy-biisochinolin, Di-(N-Oxy)-biisochinolin ausgewählt ist.

9. Komplexe nach einem der Ansprüche 1 bis 8, dadurch gekennzeichnet, daß die molekulare Einheit, die eine Triplett-Energie über der Triplett-Energie des Emissionsniveaus des komplexierten Seltenerdmetallions aufweist, und der Stickstoffheterocyclus, in dem mindestens eines der Stickstoffatome eine Oxygruppe trägt, eine einzige und identische Einheit sind.

10. Komplexe nach einem der Ansprüche 1 bis 9, dadurch gekennzeichnet, daß das Seltenerdmetallion unter Europium, Terbium, Samarium und Dysprosium ausgewählt ist.

11. Komplexe nach einem der Ansprüche 1 bis 10, dadurch gekennzeichnet, daß mindestens eine der Gruppen Ⓐ, Ⓑ, © und Ⓓ mit einer Gruppe -CO-NH-Y-Z substituiert ist, worin bedeuten

- Y eine Gruppe oder eine Spacergruppe, die aus einer zweiwertigen organischen Gruppe besteht, die unter den linearen oder verzweigten $C_{1-20}$-Alkylengruppen, die ggf. eine oder mehrere Doppelbindungen enthalten und/oder ggf. ein oder mehrere Heteroatome, wie Sauerstoff, Stickstoff, Schwefel oder Phosphor, in ihrer Kette enthalten, und den $C_{5-8}$-Cycloalkylengruppen oder den $C_{6-14}$-Arylengruppen ausgewählt ist, wobei die Alkylen-, Cycloalkylen- oder Arylengruppe ggf. mit Alkyl-, Aryl- oder Sulfonatgruppen substituiert sind und
- Z eine funktionelle Gruppe, die dazu befähigt ist, eine kovalente Bindung mit einer biologischen Substanz einzugehen.

12. Komplexe nach einem der Ansprüche 1 bis 10, dadurch gekennzeichnet, daß die makrocyclische Verbindung die Verbindung der folgenden Formel ist:

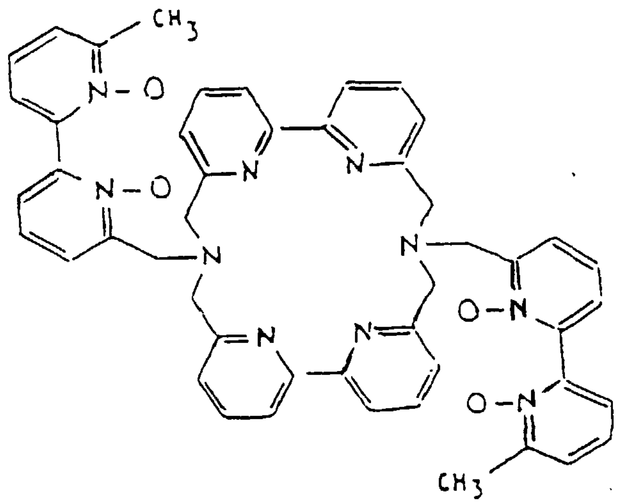

**13.** Verfahren zur Verminderung der Interferenzen bei der quantitativen Bestimmung einer zu analysierenden Substanz durch Fluoreszenz, dadurch gekennzeichnet, daß als Tracer ein makrocyclischer Komplex nach einem der Ansprüche 1 bis 12 verwendet wird.

**14.** Verfahren nach Anspruch 13, dadurch gekennzeichnet, daß das Bestimmungsmedium ein biologisches Medium ist.

**15.** Verfahren nach Anspruch 14, dadurch gekennzeichnet, daß das Bestimmungsmedium ein Serum ist.

**16.** Verwendung eines makrocyclischen Komplexes eines Seltenerdmetalls nach einem der Ansprüche 1 bis 12 zur Verminderung der Interferenzen in dem Bestimmungsmedium eines Verfahrens zur qualitativen und/oder quantitativen Bestimmung einer zu analysierenden Substanz durch Fluoreszenz in einem Medium, in dem sie enthalten sein kann.

**17.** Verwendung nach Anspruch 16 in einem homogenen Verfahren zur quantitativen und/oder qualitativen Bestimmung einer zu analysierenden Substanz durch Fluoreszenz in einem Medium, in dem sie enthalten sein kann.

**18.** Verwendung nach einem der Ansprüche 16 oder 17 in einem Verfahren zur qualitativen und/oder quantitativen Bestimmung einer zu analysierenden Substanz durch Fluoreszenz in einem Medium, in dem sie enthalten sein kann, mit folgenden Schritten:

1) Zugabe eines ersten Reaktanten, der aus mindestens einem Rezeptor der zu analysierenden Substanz besteht, zu diesem Medium,
2) Zugabe eines zweiten Reaktanten, der unter der zu analysierenden Substanz oder mindestens einem ihrer Rezeptoren ausgewählt ist, wobei einer der beiden Reaktanten mit einer fluoreszierenden Donorverbindung gekuppelt ist, die aus einem Komplex nach einem der Ansprüche 1 bis 12 besteht, und der andere Reaktant an eine fluoreszierende Akzeptorverbindung gekuppelt ist, wobei die Reihenfolge der Zugabe der Reaktanten vertauscht werden kann,
3) Inkubation des resultierenden Mediums entweder nach Zugabe jedes Reaktanten oder nach Zugabe der beiden Reaktanten,
4) Anregung des resultierenden Mediums bei der Anregungswellenlänge der Donorverbindung, und
5) Bestimmung des von der fluoreszierenden Akzeptorverbindung emittierten Signals im Gleichgewichtszustand oder als kinetische Bestimmung.

**19.** Verwendung nach Anspruch 18 in einem Verfahren zur qualitativen und/oder quantiativen Bestimmung einer zu analysierenden Substanz durch Fluoreszenz in einem Medium, in dem sie enthalten sein kann, durch ein Überschußverfahren mit folgenden Schritten

1) Zugabe eines ersten Reaktanten, der aus mindestens einem Rezeptor der zu analysierenden Substanz besteht und der an eine fluoreszierende Donorverbindung gebunden ist, die aus einem Komplex nach einem

der Ansprüche 1 bis 12 besteht, zu dem die zu analysierende Substanz enthaltenden Medium,
2) Zugabe eines zweiten Reaktanten, der aus einem oder mehreren weiteren Rezeptoren der zu analysierenden Substanz besteht, wobei der zweite Reaktant an eine fluoreszierende Akzeptorverbindung gebunden ist,
3) Inkubation des Mediums nach jeder Zugabe der Reaktanten oder nach Zugabe der beiden Reaktanten,
4) Anregung des resultierenden Mediums bei der Anregungswellenlänge der fluoreszierenden Donorverbindung mit einer Lichtquelle, und
5) Bestimmung des von der fluoreszierenden Akzeptorverbindung emittierten Signals.

**20.** Verwendung nach Anspruch 18 in einem Verfahren zur qualitativen und/oder quantitativen Bestimmung einer zu analysierenden Substanz durch Fluoreszenz in einem Medium, in dem sie enthalten sein kann, mit einem kompetitiven Verfahren mit folgenden Schritten:

1) Zugabe eines ersten Reaktanten, der aus einem Rezeptor der zu analysierenden Substanz besteht und der an eine fluoreszierende Donorverbindung gebunden ist, die aus einem Komplex nach einem der Ansprüche 1 bis 9 besteht, zu dem die zu analysierende Substanz enthaltenden Medium,
2) Zugabe eines zweiten Reaktanten, der aus der zu analysierenden Substanz besteht, die an eine fluoreszierende Akzeptorverbindung gebunden ist,
3) Inkubation des Mediums nach jeder Zugabe der Reaktanten oder nach Zugabe der beiden Reaktanten,
4) Anregung des resultierenden Mediums bei der Anregungswellenlänge der fluoreszierenden Donorverbindung, und
5) Bestimmung des von der fluoreszierenden Akzeptorverbindung emittierten Signals.

**21.** Verwendung nach Anspruch 18 in einem Verfahren zur qualitativen und/oder quantitativen Bestimmung einer zu analysierenden Substanz durch Fluoreszenz in einem Medium, in dem sie enthalten sein kann, mit einem kompetitiven Verfahren mit folgenden Schritten:

1) Zusatz eines ersten Reaktanten, der aus einem Rezeptor der zu analysierenden Substanz besteht, zu dem die zu analysierende Substanz enthaltenden Medium, wobei der Rezeptor an eine fluoreszierende Akzeptorverbindung gebunden ist,
2) Zugabe der zu analysierenden Substanz als zweiter Reaktant, die an eine fluoreszierende Donorverbindung gebunden ist, die aus einem Komplex nach einem der Ansprüche 1 bis 9 besteht,
3) Inkubation des Mediums nach Zugabe jedes Reaktanten oder nach Zugabe der beiden Reaktanten,
4) Anregung des resultierenden Mediums bei der Anregungswellenlänge der fluoreszierenden Donorverbindung, und
5) Bestimmung des von der fluoreszierenden Akzeptorverbindung emittierten Signals.

**22.** Verwendung nach einem der Ansprüche 18 bis 21, dadurch gekennzeichnet, daß der erste Reaktant und der zweite Reaktant gleichzeitig dem Medium zugesetzt werden, das die zu analysierende Substanz enthält.

**23.** Verwendung nach einem der Ansprüche 18 oder 19, dadurch gekennzeichnet, daß ein einziger Rezeptor der zu analysierenden Substanz verwendet wird, der entweder an die fluoreszierende Donorverbindung oder an die fluoreszierende Akzeptorverbindung gebunden ist.

**24.** Verwendung nach einem der Ansprüche 18 bis 23, dadurch gekennzeichnet, daß das Seltenerdmetallion des makrocyclischen Komplexes, der als fluoreszierende Donorverbindung verwendet wird, Europium ist, und dadurch, daß die fluoreszierende Akzeptorverbindung unter Allophycocyanin, Allophycocyanin B, Phycocyanin C oder Phycocyanin R ausgewählt ist.

**25.** Verwendung nach einem der Ansprüche 18 bis 23, dadurch gekennzeichnet, daß das Seltenerdmetallion des makrocyclischen Komplexes, der als fluoreszierende Akzeptorverbindung verwendet wird, Terbium ist und dadurch, daß die fluoreszierende Akzeptorverbindung unter den Rhodaminen, Thionin, Phycocyanin R, Phycoerythrocyanin, Phycoerythrin C, Phycoerythrin B oder Phycoerytrin R ausgewählt ist.

## Claims

**1.** Macrocyclic rare earth complexes characterised in that they consist of at least one rare earth salt complexed by a macrocyclic compound of formula (I):

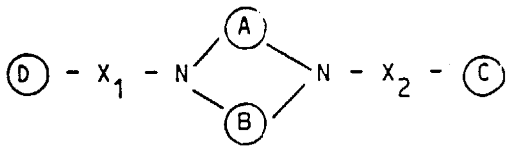

(I)

in which:

- the bivalent radicals Ⓐ, Ⓑ, ©, and Ⓓ, which are identical or different, are hydrocarbon chains optionally containing one or more heteroatoms, at least one of said radicals containing at least one molecular unit or essentially consisting of a molecular unit possessing a triplet energy greater than the energy of the emission level of the complexed rare earth ion, at least one of said radicals consisting of a substituted or unsubstituted nitrogen-containing heterocyclic system in which at least one of the nitrogen atoms carries an oxy group, and it being possible for one of the radicals © or Ⓓ not to exist;
- $X_1$ and $X_2$, which are identical or different, are hydrogen or a hydrocarbon chain $(CH_2)_n$ optionally interrupted by 1 or more heteroatoms, n being an integer from 1 to 10, with the proviso that if the radicals Ⓐ and/or Ⓑ are a nitrogen-containing heterocyclic system in which at least one of the nitrogen atoms carries an oxy group, the radicals © and/or Ⓓ are selected from biquinolines, biisoquinolines, bipyridines, terpyridines, coumarins, bipyrazines, bipyrimidines and pyridines.

**2.** The complexes according to claim 1, characterised in that at least one of the bivalent radicals Ⓐ and Ⓑ contains at least one molecular unit or essentially consists of a molecular unit possessing a triplet energy greater than the energy of the emission level of the complexed rare earth ion, and at least one of the radicals © and Ⓓ consists of a nitrogen-containing heterocyclic system in which at least one of the nitrogen atoms carries an oxy group.

**3.** The complexes according to one of claims 1 or 2, characterised in that the radicals Ⓐ and Ⓑ are identical.

**4.** The complexes according to one of claims 1 or 2, characterised in that the radicals © and Ⓓ are identical.

**5.** The complexes according to any one of claims 1 to 4, characterised in that $X_1$ and $X_2$ are identical.

**6.** The complexes according to any one of claims 1 to 5, characterised in that the triplet energy of the molecular unit is greater than 17,300 $cm^{-1}$.

**7.** The complexes according to any one of claims 1 to 6, characterised in that the molecular unit is selected from phenanthroline, anthracene, bipyridines, biquinolines, terpyridines, coumarins, bipyrazines, bipyrimidines, azobenzene, azopyridine, pyridines or 2,2'-biisoquinoline, or the units

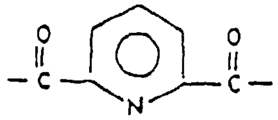

;

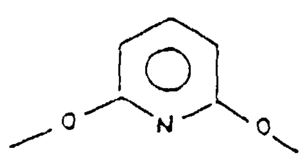

or

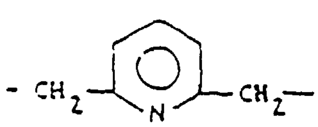

**8.** The complexes according to any one of claims 1 to 7, characterised in that the nitrogen-containing heterocyclic system is selected from the following units: pyridine N-oxide, bipyridine N-oxide, bipyridine di-N-oxide, bisisoquinoline N-oxide and bisisoquinoline di-N-oxide.

**9.** The complexes according to any one of claims 1 to 9, characterised in that the molecular unit possessing a triplet energy greater than the energy of the emission level of the complexed rare earth ion, and the nitrogen-containing heterocyclic system in which at least one of the nitrogen atoms carries an oxy group, are one and the same unit.

**10.** The complexes according to any one of claims 1 to 9, characterised in that the rare earth ion is selected from europium, terbium, samarium and dysprosium.

**11.** The complexes according to any one of claims 1 to 10, characterised in that at least one of the radicals Ⓐ, Ⓑ, © and Ⓓ is substituted by a group -CO-NH-Y-Z, in which

- Y is a spacer arm or group which consists of a bivalent organic radical selected from linear or branched $C_1$ to $C_{20}$ alkylene groups optionally containing one or more double bonds and/or optionally being interrupted by one or more heteroatoms such as oxygen, nitrogen, sulfur or phosphorus, from $C_5$-$C_8$ cycloalkylene groups or from $C_6$-$C_{14}$ arylene groups, said alkylene, cycloalkylene or arylene groups optionally being substituted by alkyl, aryl or sulfonate groups.
- Z is a functional group capable of bonding covalently with a biological substance.

**12.** The complexes according to any one of claims 1 to 10, characterised in that the macrocyclic compound is the compound of the formula

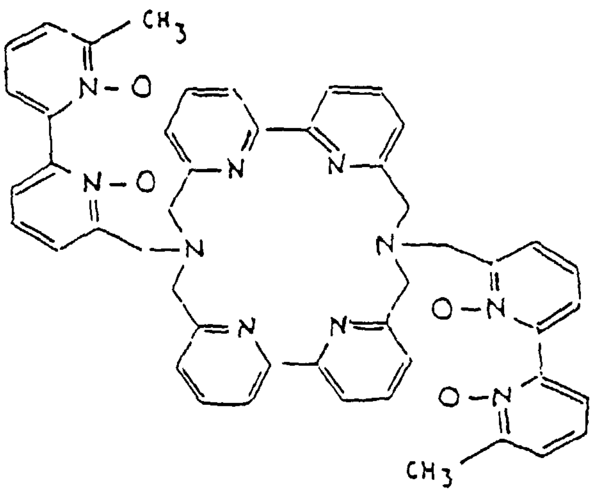

**13.** A method of reducing interference in a fluorescent assay of an analyte, characterised in that a macrocyclic complex according to any one of claims 1 to 12 is used as a tracer.

**14.** The method according to claim 13, characterised in that the measuring medium is a biological medium.

**15.** The method according to claim 14, characterised in that the measuring medium is a serum medium.

**16.** A use of a macrocyclic rare earth complex according to any one of claims 1 to 12 for reducing interference in the measuring medium of a fluorescent method of detecting and/or determining an analyte in a medium in which it may be present.

**17.** The use according to claim 16, in a homogeneous fluorescent method of detecting and/or determining an analyte in a medium in which it may be present.

**18.** The use according to one of claims 16 or 17 in a fluorescent method of detecting and/or determining an analyte in a medium in which it may be present, said method consisting in:

1) adding to said medium a first reagent consisting of at least one receptor for said analyte,
2) adding a second reagent selected from the analyte or at least one of its receptors, one of the two reagents being coupled with a fluorescent donor compound consisting of a complex according to any one of claims 1

to 12, and the other reagent being coupled with a fluorescent acceptor compound, and it being possible for the order of addition of the reagents to be reversed,
3) incubating the resulting medium either after the addition of each reagent or after the addition of both reagents,
4) exciting the resulting medium at the excitation wavelength of the donor compound,
5) measuring, at equilibrium or under kinetic conditions, the signal emitted by the fluorescent acceptor compound.

**19.** The use according to claim 18 in a fluorescent method of detecting and/or determining an analyte in a medium in which it may be present, with the aid of an excess method consisting in:

1) adding, to said medium containing the target analyte, a first reagent consisting of at least one receptor for said analyte, coupled with a fluorescent donor compound consisting of a complex according to any one of claims 1 to 12,
2) adding a second reagent consisting of one or more other receptors for said analyte, said second reagent being coupled with a fluorescent acceptor compound,
3) incubating said medium after the addition of each reagent or after the addition of both reagents,
4) exciting the resulting medium at the excitation wavelength of the fluorescent donor compound by means of a light source,
5) measuring the signal emitted by the fluorescent acceptor compound.

**20.** The use according to claim 18 in a fluorescent method of detecting and/or determining an analyte in a medium in which it may be present, with the aid of a competitive method consisting in:

1) adding, to said medium containing the target analyte, a first reagent consisting of a receptor for said analyte, coupled with a fluorescent donor compound consisting of a complex according to any one of claims 1 to 9,
2) adding a second reagent consisting of the analyte coupled with a fluorescent acceptor compound,
3) incubating said medium after the addition of each reagent or after the addition of both reagents,
4) exciting the resulting medium at the excitation wavelength of the fluorescent donor compound,
5) measuring the signal emitted by the fluorescent acceptor compound.

**21.** The use according to claim 18 in a fluorescent method of detecting and/or determining an analyte in a medium in which it may be present, with the aid of a competitive method consisting in:

1) adding, to said medium containing the target analyte, a first reagent consisting of a receptor for said analyte, said receptor being coupled with a fluorescent acceptor compound,
2) adding, as a second reagent, the analyte being coupled with a fluorescent donor compound consisting of a complex according to any one of claims 1 to 9,
3) incubating said medium either after the addition of each reagent or after the addition of both reagents,
4) exciting the resulting medium at the excitation wavelength of the fluorescent donor compound,
5) measuring the signal emitted by the fluorescent acceptor compound.

**22.** The use according to any one of claims 18 to 21, characterised in that the first reagent and second reagent are added simultaneously to the medium containing the target analyte.

**23.** The use according to one of claims 18 or 19, characterised in that a single receptor for the analyte is used which is coupled either with the fluorescent donor compound or with the fluorescent acceptor compound.

**24.** The use according to any one of claims 18 to 23, characterised in that the rare earth ion of the macrocyclic complex used as the fluorescent donor compound is europium, and in that the fluorescent acceptor compound is selected from allophycocyanin, allophycocyanin B, phycocyanin C or phycocyanin R.

**25.** The use according to any one of claims 18 to 23, characterised in that the rare earth ion of the macrocyclic complex used as the fluorescent donor compound is terbium, and in that the fluorescent acceptor compound is selected from rhodamines, thionine, phycocyanin R, phycoerythrocyanin, phycoerythrin C, phycoerythrin B or phycoerythrin R.